# EUROPEAN PATENT APPLICATION

(11) **EP 1 361 431 A1**
(43) Date of publication of application: **12.11.2003**
(21) Application number: 03009912.1
(22) Date of filing: 30.04.2003
(51) Int. Cl.: G01N 33/18

(54) **A device for measuring the degree of hardness of the water supplied to a household washing appliance**

(30) Priority: 08.05.2002 IT TO20020381
(71) Applicant: BITRON S.p.A., 10042 Nichelino (Torino) (IT)
(72) Inventor: Brignone, Enzo, 12025 Frazione Monastero Dronero (Cuneo) (IT)
(74) Representative: Gerbino, Angelo

(57) **Abstract**

The device for measuring the degree of hardness of the water supplied to a household washing appliance such as a dishwasher or a washing machine comprises a container (28) through which at least a portion of the flow of supply water is intended to pass and which is filled with ion-exchange resins (30) which can change colour as a result of the ion-exchange reaction, and a sensor (32) for detecting the colour of the resins and/or a chemical/physical property that is correlated with the colour-change of the resins (30).

## Description

The present invention relates to a device for measuring the degree of hardness of the water supplied to a household washing appliance, such as a dishwasher or a washing machine.

The object of the present invention is to provide a device of the type indicated above which ensures reliable operation and a low cost.

According to the invention, this object is achieved by means of a measuring device characterized in that it comprises a container through which at least a portion of the flow of supply water is intended to pass and which is filled with ion-exchange resins which can change colour as a result of the ion-exchange reaction, and a sensor for detecting the colour of the resins and/or a chemical/physical property that is correlated with the colour-change of the resins.

Since the colour adopted by the ion-exchange resins can be correlated with the quantity and with the degree of hardness of the water with which they have come into contact, the device of the invention can measure very precisely this hardness value, a knowledge of which is critical in order to establish particular operating parameters of the household appliance.

A further subject of the present invention is a household washing appliance such as a dishwasher or a washing machine comprising a hardness-measuring device of the type described above.

Advantages and characteristics of the present invention will become clear from the following detailed description which is provided by way of non-limiting example with reference to the appended drawings, in which:
Figure 1 is a schematic view of the main components of the water circuit of a household appliance such as a dishwasher, provided with a measuring device according to the invention,
Figure 2 is a schematic view of the main components of the water circuit of a household appliance such as a dishwasher, provided with a second embodiment of the measuring device, and
Figure 3 is a schematic view of the main components of the water circuit of a household appliance such as a dishwasher, provided with a third embodiment of the measuring device.

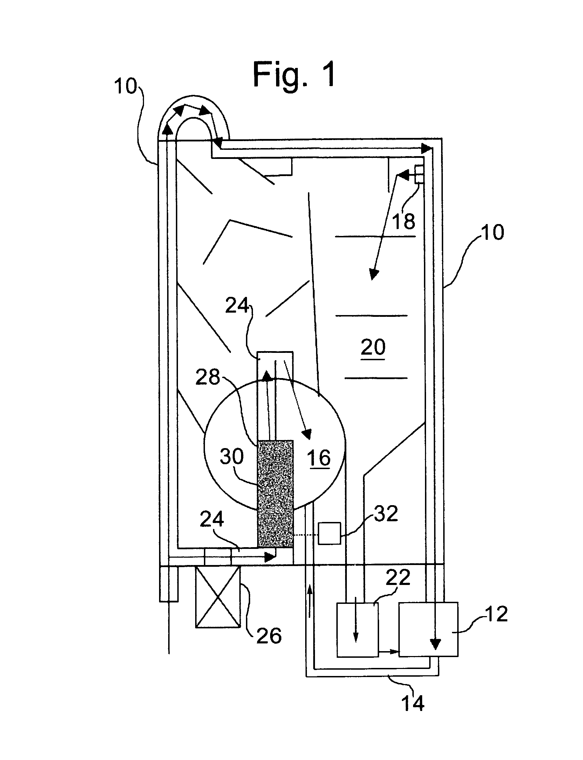

A main water-supply duct, generally indicated 10 in Figure 1, extends, in known manner, towards a decalcifying device 12 from which a decalcified-water outlet duct 14 extends towards the washing tank 16. Again in known manner, a branch 18 formed in the main duct 10 supplies a chamber 20 for collecting water to be supplied to a salt reservoir 22 so as to form brine for regenerating the decalcifying device 12.

An auxiliary duct 24 also extends from the main duct; a shut-off valve 26 and a container 28 filled with ion-exchange resins 30 which can change colour as a result of the ion-exchange reaction are located in the auxiliary duct 24. The container 28 is preferably constructed in the form of a cartridge that can be inserted in and removed from the household appliance and which has transparent walls so that the resins 30 are visible from outside the appliance.

A sensor 32, for example, of the phototransistor type, is associated with the container 28 to detect the colour and/or a chemical/physical property, such as the pH, that is correlated with the colour-change of the resins 30. The assembly constituted by the sensor 32 and the container 28 filled with resins 30 constitutes a device for measuring the degree of hardness of the water supplied to the household appliance.

Downstream of the container 28, the auxiliary duct 24 opens directly into the washing tank 16 so as to be arranged hydraulically in parallel with the main duct 10.

When the household appliance is installed in the place of use, the valve 26 is opened for the first time so that the water can flow both through the main duct 10 and through the auxiliary duct 24 whilst the first washing cycle is being performed. When the water passes through the container 28, the ions which determine its hardness react with the resins 30, changing their pH and colour which changes, for example, from green to blue. This change is measured by the sensor 32. In particular, the sensor can detect the change in pH which is brought about by the passage of a predetermined volume of water, and which can be correlated unequivocally with the value of the degree of hardness of the mains water. On the basis of this latter value, an electronic control unit can advantageously adjust the various operating parameters of the household appliance such as, for example, the volume of brine and the frequency of the process for the regeneration of the decalcifier 12, which depend on the degree of hardness. It is particularly advantageous that the control unit always causes the regeneration process to be carried out after a number of washing cycles that is established on the basis of the degree of hardness initially measured. The disadvantages inherent in known control systems, in which the carrying-out of the regeneration process depends on the degree of water hardness measured at the time in question, are thus avoided. In fact, any breakdown even of a single component of these known systems can stop the regeneration process being carried out, with consequences that are damaging to the household appliance.

Once the initial installation stage has been completed, the device for measuring the degree of hardness has completed its task. Its decalcifying capacity is in fact exhausted after a certain number of washing cycles that depends on the degree of hardness of the mains water, and the resins 30 as a whole adopt a final colour corresponding to the condition in which substitution with ions capable of producing hardness is complete. By observing the development of this final colour from the exterior, the user has confirmation that the measuring device has performed its task correctly.

If the measuring device of the invention is used in a laundry washing machine, which typically does not have a decalcifier, the facility to observe from the exterior the colour adopted by the resins in the container after the first washing cycle also gives a visual indication of the amount of detergent to use in subsequent washing cycles. In fact this amount is determined by the degree of hardness of the water supplied, an indication of which is the fraction of resins which has changed colour after the first washing cycle. The resins container is advantageously fitted on a detergent-holding tray and has indicator means - for example, a series of lamps or LEDs - for indicating the fraction of the resins which has changed colour after the first washing cycle. These indicator means consequently continue to indicate to the user the quantity of detergent to use in subsequent washes, even a long time after the first washing cycle.

The auxiliary duct 24 can in any case also be used, after the resins 30 are exhausted, to supply directly to the washing tank 16 non-decalcified water which is mixed with the decalcified water coming from the decalcifier 12. This arrangement is advantageous when the mains water has very low hardness and is therefore completely decalcified as a result of its passage through the decalcifier 12. By virtue of the mixing process just described, the hardness of the water that is present in the tank 16 is low, but not zero, so as to be optimal for the efficacy of the surfactants used.

If the site of installation of the household appliance is changed, the cartridge containing the resins 30, which by now are exhausted, can be replaced with a new cartridge and the above-described operations can be repeated to measure the degree of hardness of the mains water of the new site so that the operating parameters of the household appliance can consequently be readjusted.

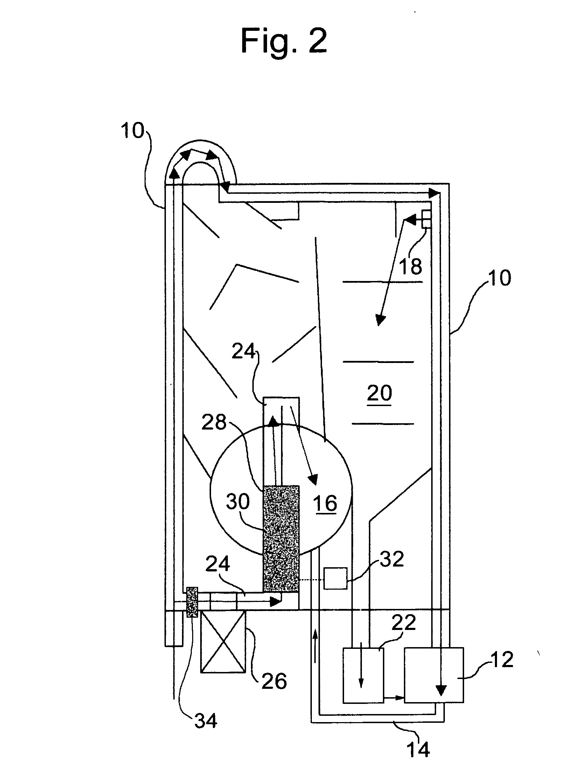

Figure 2 shows a second embodiment of the device of the invention in which the same numerals as were used with reference to Figure 1 described above again identify the same or equivalent parts.

In this embodiment, a filter 34, for example of the activated-carbon type, which can retain substances such as, for example, chlorides that are detrimental to the operation of the resins 30 is disposed in the duct 24, upstream of the container 28. The operation of the device is substantially similar to that described above.

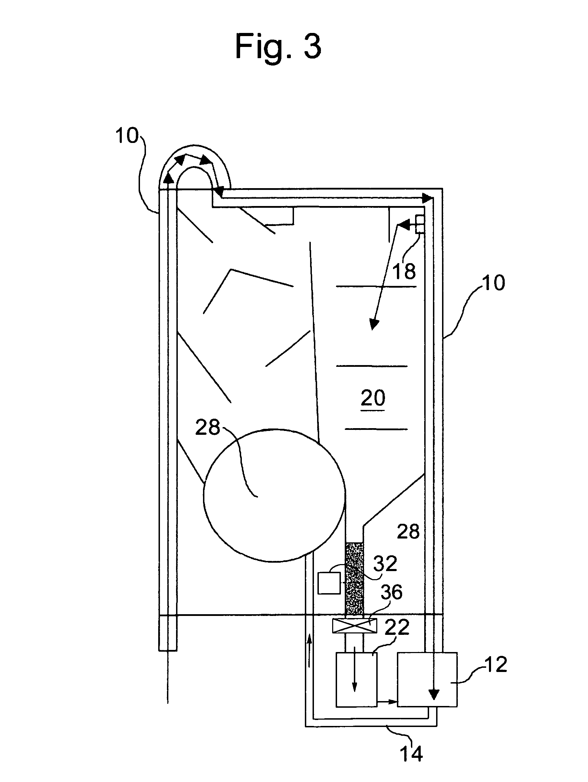

Figure 3 shows a third embodiment of the device of the invention in which the same numerals as were used with reference to Figure 1 described above again identify the same or equivalent parts.

In this embodiment, there is no auxiliary duct and the container 28 for the resins 30 is disposed in the lower portion of the chamber 20 for collecting the water to be supplied to the salt reservoir 22. A shut-off valve 36 normally used for the control of the regeneration operation is shown between the container 28 and the reservoir 22. The hardness measurement is thus performed on the water coming from the branch 18, which is collected in the chamber 20 from which it flows out through the resins 30. A multiple of the volume of the chamber 20 can advantageously be used as the predetermined reference volume of water for the hardness measurement.

Naturally, the principle of the invention remaining the same, the details of construction and forms of embodiment may be varied widely with respect to those described purely by way of example, without thereby departing from its scope.

## Claims

1. A device for measuring the degree of hardness of the water supplied to a household washing appliance such as a dishwasher or a washing machine, the device being **characterized in that** it comprises a container (28) through which at least a portion of the flow of supply water is intended to pass and which is filled with ion-exchange resins (30) which can change colour as a result of the ion-exchange reaction, and a sensor (32) for detecting the colour of the resins and/or a chemical/physical property that is correlated with the colour-change of the resins (30) .

2. A device according to Claim 1 in which the chemical/physical property that is correlated with the colour change of the resins (30) is the pH.

3. A device according to any one of the preceding claims in which the sensor (32) is of the phototransistor type.

4. A device according to any one of the preceding claims in which the container (28) filled with resins (30) is constructed in the form of a cartridge which can be inserted in and removed from the household appliance.

5. A household washing appliance such as a dishwasher or a washing machine comprising a device for measuring the degree of hardness of the water supplied, according to any one of the preceding claims.

6. A household appliance according to Claim 5 in which the container (28) filled with ion-exchange resins (30) is inserted in an auxiliary water-flow duct (24) arranged hydraulically in parallel with a main duct (10) for supplying water to a washing tank (16).

7. A household appliance according to Claim 6 in which a shut-off valve (26) is provided in the auxiliary duct (24).

8. A household appliance according to any one of Claims 5 to 7 in which the auxiliary duct (24) opens directly into the washing tank (16).

9. A household appliance according to any one of Claims 5 to 8 in which the container (28) has transparent walls so that the resins (30) are visible from the exterior.

10. A household appliance according to Claim 9 in which the container (28) is fitted in a tray for holding detergent.

11. A household appliance according to Claim 9 or Claim 10 in which the container (28) is also provided with means for indicating the fraction of resins (30) which has changed colour after the first washing cycle.

12. A household appliance according to any one of Claims 5 to 11 in which a filter (34) which can retain substances that are detrimental to the operation of the resins (30) is located upstream of the container (28).

13. A household appliance according to Claim 5 in which the container (28) filled with ion-exchange resins (30) is disposed in the lower portion of a chamber (20) for collecting water to be supplied to a reservoir (22) for holding salt.

14. A household appliance according to Claim 13 in which, in order to measure the degree of hardness, reference is made to a volume of water equal to a multiple of the volume of the chamber (20).

15. A household appliance according to any one of Claims 5 to 14 in which a control unit automatically causes the process for the regeneration of a decalcifier (12) to be carried out after a number of washing cycles that is established on the basis of the degree of hardness initially measured.
